(19) Europäisches Patentamt
European Patent Office
Office européen des brevets

(11) Veröffentlichungsnummer: **0 086 982**
**A1**

(12) **EUROPÄISCHE PATENTANMELDUNG**

(21) Anmeldenummer: **83100680.4**

(22) Anmeldetag: **26.01.83**

(51) Int. Cl.³: **C 07 D 495/04**
C 07 D 519/00, A 61 K 31/55
//(C07D495/04, 333/00, 243/00),
(C07D519/00, 495/00, 451/00)

(30) Priorität: **06.02.82 DE 3204153**

(43) Veröffentlichungstag der Anmeldung:
**31.08.83 Patentblatt 83/35**

(84) Benannte Vertragsstaaten:
**AT BE CH DE FR GB IT LI LU NL SE**

(71) Anmelder: **Dr. Karl Thomae GmbH**
**Postfach 1755**
**D-7950 Biberach (Riss)(DE)**

(72) Erfinder: **Schmidt, Günther, Dr. Dipl.-Chem.**
**Johann-Seb.-Bach-Strasse 27**
**D-7950 Biberach 1(DE)**

(72) Erfinder: **Eberlein, Wolfgang, Dr. Dipl.-Chem.**
**Obere Au 6**
**D-7950 Biberach 1(DE)**

(72) Erfinder: **Engel, Wolfhard, Dr. Dipl.-Chem.**
**Mozartstrasse 13**
**D-7950 Biberach 1(DE)**

(72) Erfinder: **Trummlitz, Günter, Dr. Dipl.-Chem.**
**Buchenweg 27**
**D-7951 Warthausen(DE)**

(72) Erfinder: **Hammer, Rudolf, Dr.**
**Via Fabio Filzi 33**
**Milano(IT)**

(72) Erfinder: **Del Soldato, Piero, Dr.**
**Via E. Toti 22**
**Monza(IT)**

(54) **Substituierte Thienobenzodiazepinone, Verfahren zu ihrer Herstellung und sie enthaltende Arzneimittel.**

(57) Beschrieben werden neue substituierte Thienobenzodiazepinone der allgemeinen Formel

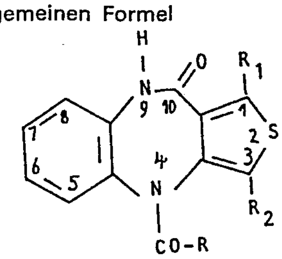

(I),

worin

$R_1$ ein Wasserstoffatom oder einen Alkylrest mit 1 bis 4 Kohlenstoffatomen bedeutet,

$R_2$ ein Halogenatom darstellt oder eine der Bedeutungen von $R_1$ hat und

R einen - im heterocyclischen Sechsring gegebenenfalls durch eine weitere Methylgruppe substituierten - (1-Methyl-4-piperidinyl)methyl-, (1-Methyl-1,2,5,6-tetrahydro-4-pyridinyl) methyl-, 1-Methyl-1,2,5,6-tetrahydro-4-pyridinyl-, (1-Methyl-4-piperidinyliden)methyl- oder (8-Methyl-8-azabicyclo[3,2,1]oct-3-yl)methyl-Rest bedeutet, sowie ihre Säureadditionssalze und Verfahren zur Herstellung dieser Verbindungen, des weiteren Arzneimittel, die die Verbindungen der allgemeinen Formel I enthalten.

Die neuen Verbindungen zeigen eine sehr gute antiulcerogene magensäuresekretionshemmende Wirkung, wobei unerwünschte Nebenwirkungen weitgehend reduziert sind.

EP 0 086 982 A1

DR. KARL THOMAE GMBH
D-7950 Biberach 1

Case 5/850
Dr.Bu/pf

# Substituierte Thienobenzodiazepinone, Verfahren zu ihrer Herstellung und sie enthaltende Arzneimittel

Die Erfindung betrifft neue substituierte Thienobenzodiazepinone, Verfahren zu ihrer Herstellung und sie enthaltende Arzneimittel.

In der deutschen Offenlegungsschrift DE-OS 1 795 176 werden bestimmte Dibenzodiazepinone mit einer ulkushemmenden und sekretionshemmenden Wirkung beschrieben. Aus der US-Patentschrift US-PS 3.953.430 sind substituierte Dibenzodiazepine mit antidepressiver und analgetischer Wirkung bekannt. In der US-PS 4.168.269 werden substituierte Thienobenzodiazepine mit analgetischer Wirkung beschrieben. Darüber hinaus werden in der europäischen Patentanmeldung EP-OS 0 039 519 Thienobenzodiazepinone mit antiulcerogenen und sekretionshemmenden Effekten erwähnt. Es wurden nun Thienobenzodiazepinone mit neuartigen Aminoacylresten gefunden, die gegenüber den oben erwähnten Verbindungen interessante und überlegene pharmakologische Wirkungen aufweisen.

Gegenstand der Erfindung sind substituierte Thienobenzodiazepinone der allgemeinen Formel I

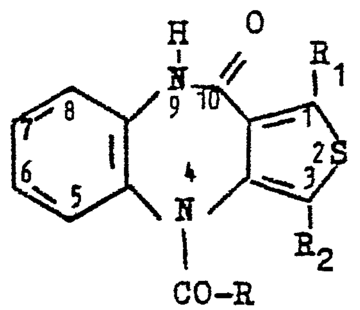

(I),

worin

$R_1$ ein Wasserstoffatom oder einen Alkylrest mit 1 bis 4 Kohlenstoffatomen bedeutet,

$R_2$ ein Halogenatom darstellt oder eine der Bedeutungen von $R_1$ hat und

R einen - im heterocyclischen Sechsring gegebenenfalls durch eine weitere Methylgruppe substituierten - (1-Methyl-4-piperidinyl)methyl-, (1-Methyl-1,2,5,6-tetrahydro-4-pyridinyl)methyl-, 1-Methyl-1,2,5,6-tetrahydro-4-pyridinyl-, (1-Methyl-4-piperidinyliden)methyl- oder (8-Methyl-8-azabicyclo[3,2,1]oct-3-yl)methyl-Rest bedeutet, sowie ihre Säureadditionssalze.

Alkylreste mit 1 bis 4 Kohlenstoffatomen sind der Methyl-, Ethyl-, Propyl-, Isopropyl-, n-Butyl-, Isobutyl-, sek.-Butyl-, tert.-Butylrest.

Besonders bevorzugt als Alkylrest $R_1$ und $R_2$ ist der Methylrest.

Halogenatome $R_2$ sind das Bromatom, insbesondere das Chloratom.

Besonders bevorzugt sind Verbindungen der allgemeinen Formel I, worin

$R_1$ ein Wasserstoffatom oder die Methylgruppe,

$R_2$ das Chloratom bedeutet oder eine der Bedeutungen von $R_1$ hat und

$\underline{R}$ einen (1-Methyl-4-piperidinyl)methyl-, (1-Methyl-1,2,5,6-tetrahydro-4-pyridinyl)methyl-, 1-Methyl-1,2,5,6-tetrahydro-4-pyridinyl-, (8-Methyl-8-azabicyclo/3,2,1/oct-3-yl)-methyl- oder (1-Methyl-4-piperidinyliden)methyl-Rest bedeutet, und/oder ihre pharmakologisch verträglichen Säureadditonssalze.

Die Verbindungen der allgemeinen Formel I können nach Umsetzung mit anorganischen oder organischen Säuren auch in Form ihrer physiologisch verträglichen Salze vorliegen. Als Säuren haben sich hierzu beispielsweise Salzsäure, Bromwasserstoffsäure, Schwefelsäure, Methylschwefelsäure, Phosphorsäure, Weinsäure, Fumarsäure, Zitronensäure, Maleinsäure, Bernsteinsäure, Gluconsäure, Äpfelsäure, p-Toluolsulfonsäure, Methansulfonsäure oder Amidosulfonsäure als geeignet erwiesen.

Zur Erläuterung des Erfindungsgegenstandes seien als Beispiele folgende Verbindungen genannt:

4,9-Dihydro-4-/(1-methyl-4-piperidinyl)acetyl/-10H-thieno/3,4-b//1,5/benzodiazepin-10-on

4,9-Dihydro-4-/(1,3-dimethyl-4-piperidinyl)acetyl/-10H-thieno/3,4-b//1,5/benzodiazepin-10-on

4,9-Dihydro-4-/(1,2-dimethyl-4-piperidinyl)acetyl/-10H-thieno/3,4-b//1,5/benzodiazepin-10-on

4,9-Dihydro-1-methyl-4-/(1-methyl-4-piperidinyl)acetyl/-10H-thieno/3,4-b//1,5/benzodiazepin-10-on

4,9-Dihydro-3-methyl-4-[(1-methyl-4-piperidinyl)acetyl]-10H-thieno[3,4-b][1,5]benzodiazepin-10-on

4,9-Dihydro-1,3-dimethyl-4-[(1-methyl-4-piperidinyl)acetyl-10H-thieno[3,4-b][1,5]benzodiazepin-10-on

3-Chlor-4,9-dihydro-4[(1-methyl-4-piperidinyl)acetyl]-10H-thieno[3,4-b][1,5]benzodiazepin-10-on

4,9-Dihydro-4-[(1-methyl-1,2,5,6-tetrahydro-4-pyridinyl)acetyl]-10H-thieno[3,4-b][1,5]benzodiazepin-10-on

4,9-Dihydro-4-[(1,2-dimethyl-1,2,5,6-tetrahydro-4-pyridinyl)-acetyl]-10H-thieno[3,4-b][1,5]benzodiazepin-10-on

4,9-Dihydro-4-[(1,3-dimethyl-1,2,5,6-tetrahydro-4-pyridinyl)-acetyl]-1-methyl-10H-thieno[3,4-b][1,5]benzodiazepin-10-on

4,9-Dihydro-4-[(1,5-dimethyl-1,2,5,6-tetrahydro-4-pyridinyl)-acetyl]-10H-thieno[3,4-b][1,5]benzodiazepin-10-on

4,9-Dihydro-4-[(1,6-dimethyl-1,2,5,6-tetrahydro-4-pyridinyl)-acetyl]-10H-thieno[3,4-b][1,5]benzodiazepin-10-on

4,9-Dihydro-1-methyl-4-[(1-methyl-1,2,5,6-tetrahydro-4-pyridinyl)acetyl]-10H-thieno[3,4-b][1,5]benzodiazepin-10-on

4,9-Dihydro-3-methyl-4-[(1-methyl-1,2,5,6-tetrahydro-4-pyridinyl)acetyl]-10H-thieno[3,4-b][1,5]benzodiazepin-10-on

4,9-Dihydro-1,3-dimethyl-4-[(1-methyl-1,2,5,6-tetrahydro-4-pyridinyl)acetyl]-10H-thieno[3,4-b][1,5]benzodiazepin-10-on

3-Chlor-4,9-dihydro-4-[(1-methyl-1,2,5,6-tetrahydro-4-pyridinyl)acetyl]-10H-thieno[3,4-b][1,5]benzodiazepin-10-on

4,9-Dihydro-4-[(1-methyl-1,2,5,6-tetrahydro-4-pyridinyl)-carbonyl]-10H-thieno[3,4-b][1,5]benzodiazepin-10-on

4,9-Dihydro-4-[(1,2-dimethyl-1,2,5,6-tetrahydro-4-pyridinyl)-carbonyl]-10H-thieno[3,4-b][1,5]benzodiazepin-10-on

4,9-Dihydro-4-[(1,3-dimethyl-1,2,5,6-tetrahydro-4-pyridinyl)-carbonyl]-10H-thieno[3,4-b][1,5]benzodiazepin-10-on

4,9-Dihydro-4-[(1,5-dimethyl-1,2,5,6-tetrahydro-4-pyridinyl)-carbonyl]-1-methyl-10H-thieno[3,4-b][1,5]benzodiazepin-10-on

4,9-Dihydro-4-[(1,6-dimethyl-1,2,5,6-tetrahydro-4-pyridinyl)-carbonyl]-10H-thieno[3,4-b][1,5]benzodiazepin-10-on

4,9-Dihydro-1-methyl-4-[(1-methyl-1,2,5,6-tetrahydro-4-pyridinyl)carbonyl]-10H-thieno[3,4-b][1,5]benzodiazepin-10-on

4,9-Dihydro-3-methyl-4-[(1-methyl-1,2,5,6-tetrahydro-4-pyridinyl)carbonyl]-10H-thieno[3,4-b][1,5]benzodiazepin-10-on

4,9-Dihydro-1,3-dimethyl-4-[(1-methyl-1,2,5,6-tetrahydro-4-pyridinyl)carbonyl]-10H-thieno[3,4-b][1,5]benzodiazepin-10-on

3-Chlor-4,9-dihydro-4-[(1-methyl-1,2,5,6-tetrahydro-4-pyridinyl)carbonyl]-10H-thieno[3,4-b][1,5]benzodiazepin-10-on

endo-4,9-Dihydro-4-[(8-methyl-8-azabicyclo[3,2,1]oct-3-yl)-acetyl]-10H-thieno[3,4-b][1,5]benzodiazepin-10-on

exo-4,9-Dihydro-4-[(8-methyl-8-azabicyclo[3,2,1]oct-3-yl)-acetyl]-10H-thieno[3,4-b][1,5]benzodiazepin-10-on

endo-4,9-Dihydro-1-methyl-4-[(8-methyl-8-azabicyclo[3,2,1]-oct-3-yl)acetyl]-10H-thieno[3,4-b][1,5]benzodiazepin-10-on

exo-4,9-Dihydro-1-methyl-4-[(8-methyl-8-azabicyclo[3,2,1]-oct-3-yl)acetyl]-10H-thieno[3,4-b][1,5]benzodiazepin-10-on

endo-4,9-Dihydro-3-methyl-4-[(8-methyl-8-azabicyclo[3,2,1]-oct-3-yl)acetyl]-10H-thieno-[3,4-b][1,5]benzodiazepin-10-on

endo-4,9-Dihydro-1,3-dimethyl-4-[(8-methyl-8-azabicyclo-[3,2,1]oct-3-yl)acetyl]-10H-thieno[3,4-b][1,5]benzodiazepin-10-on

endo-3-Chlor-4,9-dihydro-4-[(8-methyl-8-azabicyclo[3,2,1]-oct-3-yl)acetyl]-10H-thieno[3,4-b][1,5]benzodiazepin-10-on

4,9-Dihydro-4-[(1-methyl-4-piperidinyliden)acetyl]-10H-thieno-[3,4-b][1,5]benzodiazepin-10-on

4,9-Dihydro-1-methyl-4-[(1-methyl-4-piperidinyliden)acetyl]-10H-thieno[3,4-b][1,5]benzodiazepin-10-on

4,9-Dihydro-3-methyl-4-[(1-methyl-4-piperidinyliden)acetyl]-10H-thieno[3,4-b][1,5]benzodiazepin-10-on

4,9-Dihydro-1,3-dimethyl-4-[(1-methyl-4-piperidinyliden)-acetyl]-10H-thieno[3,4-b][1,5]benzodiazepin-10-on

3-Chlor-4,9-dihydro-4-[(1-methyl-4-piperidinyliden)acetyl]-10H-thieno[3,4-b][1,5]benzodiazepin-10-on

Die substituierten Thienobenzodiazepinone der allgemeinen Formel I und ihre Säureadditionssalze besitzen wertvolle Eigenschaften, die sie gewerblich verwertbar machen, insbesondere sind sie durch eine ausgezeichnete Magen- und Darmschutzwirkung bei Warmblütern gekennzeichnet, sie hemmen z.B. die Bildung von Magengeschwüren. Ferner weisen sie, bedingt durch eine geringe Toxizität und das Fehlen wesentlicher Nebenwirkungen eine günstige therapeutische Breite auf.

Die ausgezeichnete Wirksamkeit der pharmakologisch wirksamen substituierten Thienobenzodiazepinone der allgemeinen Formel I und ihrer pharmakologisch. d.h. biologisch verträglichen Säureadditionssalze ermöglicht ihren Einsatz in der Human- und auch in der Veterinärmedizin, wobei sie zur Behandlung und Prophylaxe von Krankheiten, die auf Erkrankungen des Magens oder Darms beruhen, verwendet werden. Beispielsweise können mit ihnen akutes und chronisches Ulcus ventriculi und Ulcus duodeni, Gastritis oder hyperacider Reizmagen bei Mensch oder Tier behandelt werden.

Die Verbindungen der allgemeinen Formel I lassen sich in an sich bekannter Weise in die üblichen pharmazeutischen Zubereitungsformen, z.B. in Lösungen, Suppositorien, Tabletten, Dragees, Kapseln oder Teezubereitungen einarbeiten.

Die Tagesdosis bei oraler Gabe liegt im allgemeinen zwischen 0,01 und 5, vorzugsweise 0,02 und 2,5, insbesondere 0,05 und 1,0 mg/kg Körpergewicht, die gegebenenfalls in Form mehrerer, vorzugsweise 1 bis 3 Einzelgaben zur Erzielung der gewünschten Ergebnisse verabreicht wird.

Ein weiterer Gegenstand der Erfindung sind also Arzneimittel, welche die Verbindungen der allgemeinen Formel I enthalten. Ebenso umfaßt die Erfindung die Verwendung der erfindungsgemäßen Verbindungen bei der Herstellung von Arzneimitteln, die zur Bekämpfung der angeführten Krankheiten eingesetzt werden.

Sollen die erfindungsgemäßen substituierten Thienobenzodiazepinone der allgemeinen Formel I und/oder ihre pharmakologisch verträglichen Säureadditionssalze zur Behandlung der angegebenen Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat; Sektretionshemmer, wie $H_2$-Blocker, z.B. Cimetidin, Ranitidin;

Magen- und Darmtherapeutika, z.B. Metoclopramid, Bromoprid, Tiaprid; Tranquilizer, wie Benzodiazepine, beispielsweise Diazepam, Oxazepam; Spasmolytika, z.B. Bietamiverin, Camylofin; Anticholinergica, z.B. Oxyphencyclimin, Phencarbamid; Glucocorticoide, wie Prednisolon, Fluocortolon, Betamethason; nichtsteroidale Antiphlogistika, wie Arylessigsäuren und -propionsäuren, Heteroarylessigsäuren und -propionsäuren, Benzothiazincarboxamiddioxide, Pyrazolidindione, Chinazolinone, z.B. Ibuprofen, Naproxen, Diclofenac, Fenbufen, Flurbiprofen, Indometacin, Lonazolac, Sudoxicam, Piroxicam, Phenylbutazon, Bumadizon-Calcium, Proquazon; Lokalanaesthetika, beispielsweise Tetracain, Procain; gegebenenfalls auch Fermente, Vitamine, Aminosäuren etc. enthalten.

Ein weiterer Gegenstand der Erfindung sind Verfahren zur Herstellung der substituierten Thienobenzodiazepinone der allgemeinen Formel I

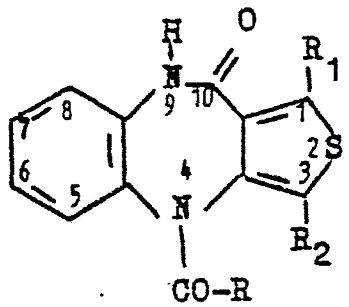

(I),

worin

$R_1$ ein Wasserstoffatom oder einen Alkylrest mit 1 bis 4 Kohlenstoffatomen bedeutet,

$R_2$ ein Halogenatom darstellt oder eine der Bedeutungen von $R_1$ hat und

R einen - im heterocyclischen Sechsring gegebenenfalls durch eine weitere Methylgruppe substituierten -

(1-Methyl-4-piperidinyl)methyl-, (1-Methyl-1,2,5,6-tetrahydro-4-pyridinyl)methyl-, 1-Methyl-1,2,5,6-tetrahydro-4-pyridinyl-, (8-Methyl-8-azabicyclo[3,2,1]oct-3-yl)methyl- oder (1-Methyl-4-piperidinyliden)methyl-Rest bedeutet, sowie von deren Säureadditionssalzen. Diese Verbindungen lassen sich wie folgt darstellen:

a) Alle Verbindungen der allgemeinen Formel I können dadurch erhalten werden, daß man Thienobenzodiazepinone der allgemeinen Formel II

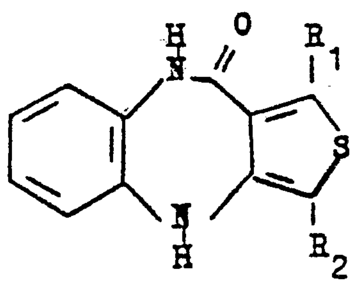

(II),

worin
$R_1$ und $R_2$ die oben angegebenen Bedeutungen haben, mit Säurederivaten der allgemeinen Formel III

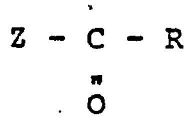

(III),

worin R die oben angegebene Bedeutung hat und Z eine nucleofuge Gruppe bzw. Abgangsgruppe darstellt, acyliert.

Die Umsetzung der Verbindungen der allgemeinen Formel II mit den Säurederivaten der allgemeinen Formel III erfolgt in an sich bekannter Weise. Die Abgangsgruppe Z ist eine Gruppe, die gemeinsam mit der Carbonylgruppe, an die sie gebunden ist, ein reaktives Carbonsäurederivat bildet. Als reaktive Carbonsäurederivate seien beispielsweise

Säurehalogenide, -ester, -anhydride oder gemischte -anhydride, wie sie aus Salzen der entsprechenden Säure (Z=OH) und Säurechloriden, wie Phosphoroxidchlorid, Diphosphorsäuretetrachlorid oder Chlorameisensäureester, gebildet werden oder die bei der Umsetzung von III (Z=OH) mit N-Alkyl-2-halogenpyridiniumsalzen entstehenden N-Alkyl-2-acyloxypyridiniumsalze genannt.

Bevorzugt wird die Reaktion mit den gemischten Anhydriden starker Mineralsäuren, insbesondere der Dichlorphosphorsäure, durchgeführt. Die Reaktion wird gegebenenfalls in Gegenwart eines säurebindenden Mittels (Protonenakzeptors) durchgeführt. Als geeignete Protonenakzeptoren seien beispielsweise genannt Alkalimetallcarbonate oder -hydrogencarbonate, wie Natriumcarbonat oder Kaliumhydrogencarbonat; tertiäre organische Amine, wie Pyridin, Triethylamin, Ethyldiisopropylamin, 4-Dimethylaminopyridin; oder Natriumhydrid. Die Reaktion wird bei Temperaturen zwischen -25$^{o}$ und 130$^{o}$C in einem inerten Lösungsmittel durchgeführt. Als inerte Lösungsmittel kommen beispielsweise in Frage chlorierte aliphatische Kohlenwasserstoffe, wie Methylenchlorid, 1,2-Dichlorethan; offenkettige oder cyclische Ether, wie Diethylether, Tetrahydrofuran oder 1,4-Dioxan; aromatische Kohlenwasserstoffe, wie Benzol, Toluol, Xylol, o-Dichlorbenzol; polare aprotische Lösungsmittel wie Acetonitril, Dimethylformamid oder Hexamethylphosphorsäuretriamid; oder Gemische davon. Die Reaktionszeiten betragen je nach Menge und Art des eingesetzten Acylierungsmittels der allgemeinen Formel III zwischen 15 Minuten und 80 Stunden. Es ist nicht nötig, die Verbindungen der allgemeinen Formel III in reiner Form herzustellen, sie können vielmehr im Reaktionsansatz in bekannter Weise in situ erzeugt werden.

b) Solche Verbindungen der allgemeinen Formel I, worin R einen im heterocyclischen Sechsring gegebenenfalls durch eine weitere Methylgruppe substituierten (1-Methyl-1,2,5,6-tetrahydro-4-pyridinyl)methyl- oder 1-Methyl-1,2,5,6-tetrahydro-4-pyridinyl-Rest bedeutet, werden auch dadurch erhalten, daß man ein Thienobenzodiazepinon der allgemeinen Formel II mit Acylierungsmitteln der allgemeinen Formel IV

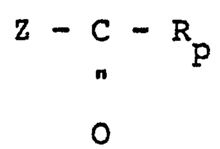

(IV),

in der Z die oben für die Formel II angegebenen Bedeutungen hat und $R_p$ ein gegebenenfalls methylsubstituierter 4-Pyridinyl- oder (4-Pyridinyl)methyl-Rest ist, zur Reaktion bringt.

Die Acylierung gelingt unter den bei a) ausgeführten Bedingungen, jedoch wird die Umsetzung in siedendem Dioxan in Gegenwart von Pyridin, 4-Dimethylamino-pyridin oder Triethylamin bevorzugt.

Die so erhaltenen Zwischenverbindungen der allgemeinen Formel V

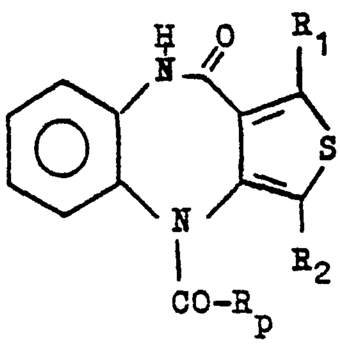

(V),

worin

$R_1$, $R_2$ und $R_p$ die oben angegebenen Bedeutungen haben, werden anschließend mit Methylierungsmitteln der allgemeinen Formel VI

$$H_3C - X \qquad (VI),$$

worin X den Säurerest von starken Sauerstoffsäuren, beispielsweise von Schwefelsäure, Methylschwefelsäure, Fluorsulfonsäure, Trifluormethansulfonsäure, Methansulfonsäure, Benzolsulfonsäure, p-Toluolsulfonsäure, p-Brombenzolsulfonsäure, Phosphorsäure oder auch Halogenid, bevorzugt Chlorid, Bromid, Jodid bedeutet,

zu Pyridiniumsalzen der allgemeinen Formel Va

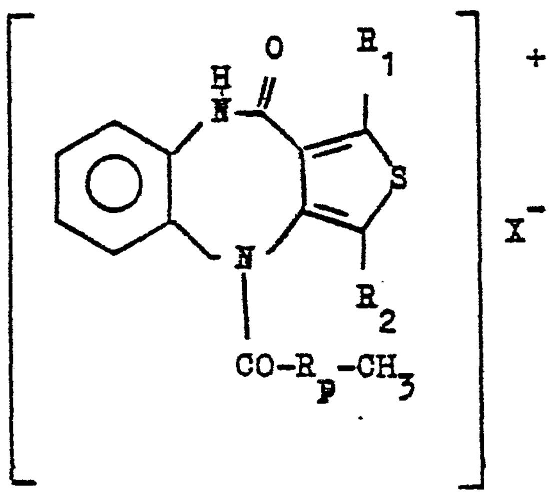

(Va),

worin $R_1$, $R_2$, $R_p$ und X die vorstehend genannten Bedeutungen haben, methyliert. Die Methylierung wird in inerten Lösungsmitteln, z.B. chlorierten aliphatischen Kohlenwasserstoffen, wie Methylenchlorid, 1,2-Dichlorethan, offenkettigen oder cyclischen Ethern, wie Diethylether oder Tetrahydrofuran,

aromatischen Kohlenwasserstoffen, wie Benzol, Toluol, Xylol oder Dichlorbenzol, bevorzugt jedoch in Dioxan, Acetonitril oder Dimethylformamid und bei Temperaturen zwischen -20 und + 130°C, bevorzugt + 30 und 100°C durchgeführt.

Anschließende Reduktion der Pyridiniumsalze Va mit Natrium - oder Kaliumtetrahydridoborat oder Natrium- oder Kaliumalkoxy-, dialkoxy- oder-trialkoxyborhydrid in protischen Lösungsmitteln, beispielsweise in Wasser, Methanol, Ethanol, 2-Propanol oder in Gemischen davon bei Temperaturen zwischen -40 und +50°C, bevorzugt bei 0°C führt zu den gesuchten Thienobenzodiazepinonen der allgemeinen Formel I, in der $R_1$ und $R_2$ die eingangs definierten Bedeutungen haben und R einen (1-Methyl-1,2,5,6-tetrahydro-4-pyridinyl)methyl- oder 1-Methyl-1,2,5,6-tetrahydro-4-pyridinyl-Rest bedeutet.

Die Verfahren zur Herstellung der pharmakologisch wirksamen Thienobenzodiazepinone der allgemeinen Formel I sind also dadurch gekennzeichnet, daß man Thienobenzodiazepinone der allgemeinen Formel II mit Verbindungen der allgemeinen Formel III acyliert oder mit Pyridinalkansäurederivaten der allgemeinen Formel IV umsetzt, anschließend methyliert und mit Borhydriden oder Alkoxyborhydriden reduziert und jeweils gegebenenfalls anschließend die erhaltene Base in ein pharmakologisch verträgliches Säureadditionssalz oder ein erhaltenes Säureadditionssalz in die freie Base oder ein pharmakologisch verträgliches Säureadditionssalz nach an sich bekannten Methoden überführt.

Ein Teil der erfindungsgemäßen Thienobenzodiazepinone der allgemeinen Formel I enthält ein oder zwei asymmetrische Kohlenstoffatome im Rest R. Diese Verbindungen können deshalb in zwei diastereomeren cis- und trans-Formen oder jeweils als enantiomere (+) - und (-)-Formen auftreten. Die Erfindung umfaßt die einzelnen Isomeren ebenso wie ihre Gemische.

Die Trennung der jeweiligen Diastereomeren gelingt auf Grund der unterschiedlichen physiko-chemischen Eigenschaften, z.B. durch fraktioniertes Umkristallisieren aus geeigneten Lösungsmitteln oder durch chromatographische Verfahren.

Die Spaltung eventueller Razemate der Verbindungen der allgemeinen Formel I kann nach bekannten Verfahren durchgeführt werden, beispielsweise unter Verwendung einer optisch aktiven Säure, wie (+)- oder (-)-Weinsäure, oder eines Derivats davon, wie (+)- oder (-)-Diacetylweinsäure, (+) oder (-)-Monomethyltartrat oder (+)-Camphersulfonsäure.

Nach einem üblichen Verfahren zur Isomerentrennung wird das Razemat einer Verbindung der allgemeinen Formel I mit einer der vorstehend beschriebenen optisch aktiven Säuren in äquimolarer Menge in einem Lösungsmittel umgesetzt und die erhaltenen kristallinen optisch aktiven Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50:50, verwendet. Sodann wird jedes der optisch aktiven Salze in Wasser gelöst, neutralisiert und dadurch die entsprechende freie Verbindung in der (+) oder (-)-Form erhalten.

Die Herstellung der Ausgangsverbindungen der allgemeinen Formel II erfolgt gemäß US-PS 3 953 430 durch Umsetzung von o-Phenylendiamin mit einem Tetrahydrothiophencarbonsäurederivat der allgemeinen Formel

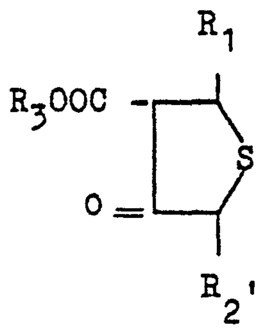

(VII),

in der $R_1$ wie oben definiert ist, $R_2'$ ein Wasserstoffatom oder eine Alkylgruppe mit 1 bis 4 Kohlenstoffatomen und $R_3$ ein Wasserstoffatom oder eine Alkylgruppe mit 1 bis 5 Kohlenstoffatomen bedeuten. Die Umsetzung erfolgt in einem inerten Lösungsmittel, wie Toluol, bei Temperaturen bis zum Siedepunkt des Reaktionsgemisches. Es entsteht dabei ein Tetrahydrothienobenzodiazepinon der allgemeinen Formel

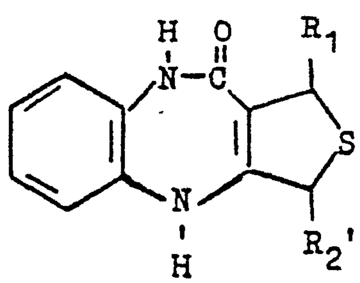

(VIII),

welches anschließend mit einem Dehydrierungsmittel, z.B. N-Bromsuccinimid in Dimethylformamid, zu den entsprechenden Verbindungen der allgemeinen Formel II, worin $R_2$ die Bedeutung $R_2'$ besitzt, umgesetzt wird. Soll daraus eine Verbindung der allgemeinen Formel II, hergestellt werden, in der $R_2$ die Bedeutung eines Halogenatoms besitzt, so wird eine Verbindung der allgemeinen Formel II, in der $R_2$ die Bedeutung eines Wasserstoffatoms hat, anschließend halogeniert nach an sich üblichen Methoden.

So werden beispielsweise erhalten:

3-Chlor-4,9-dihydro-10H-thieno[3,4-b][1,5]benzodiazepin-10-on;

4,9-Dihydro-3-methyl-10H-thieno[3,4-b][1,5]benzodiazepin-10-on; F. 228 - 230°C (Methanol);

4,9-Dihydro-1,3-dimethyl-10H-thieno[3,4-b][1,5]benzodiazepin-10-on, F. 195 - 196°C;

4,9-Dihydro-1-methyl-10H-thieno[3,4-b][1,5]benzodiazepin-10-on, F. 274 - 276°C (n-Propanol).

Verbindungen der allgemeinen Formel III und IV sind bekannt oder können, wie oben angegeben, in Analogie zu bekannten Herstellungsverfahren leicht erhalten werden. Beispielsweise erhält man bei der Umsetzung von 4-Hydroxy-1-methyl-4-piperidinessigsäure-natriumsalz mit Thionylchlorid ein Gemisch von 1-Methyl-1,2,5,6-tetrahydro-4-pyridinessigsäurechlorid und (1-Methyl-4-piperidinyliden)acetylchlorid, das ohne Trennung nach Verfahren a) mit einem Thienobenzodiazepin der allgemeinen Formel II zu einem Gemisch der gesuchten Verbindungenn der Formel I umgesetzt werden kann, worin R den (1-Methyl-1,2,5,6-tetrahydro-4-pyridinyl)methyl- und (1-Methyl-4-piperidinyliden)methyl-Rest bedeutet. Dieses Gemisch von Doppelbindungsisomeren kann anschließend gewünschtenfalls nach üblichen Verfahren, beispielsweise durch fraktionierte Kristallisation, Säulenchromatographie oder Hochdruckflüssigkeitschromatographie in seine Bestandteile zerlegt werden.

Wie bereits oben erwähnt, weisen die neuen Verbindungen der allgemeinen Formel I wertvolle pharmakologische Eigenschaften auf; insbesondere besitzen sie antiulcerogene und magensäuresekretionshemmende Wirkungen, sowie günstige Effekte auf verschiedene andere Erkrankungen des Magen-Darm-Traktes, unter denen das Colon irritabile besonders hervorgehoben sei.

Eine günstige Relation zwischen antiulcerogenen und antisekretorischen Wirkungen einerseits und den vor allem bei Therapeutika mit anticholinerger Wirkkomponente auftretenden unerwünschten Wirkungen auf die Pupillenweite und Tränen- und Speichelsekretion andererseits ist für die therapeutische Verwendung der Substanzen von besonderer Wichtigkeit. Die folgenden Versuche zeigen, daß die erfindungsgemäßen Verbindungen diesbezüglich überraschend günstige Relationen aufweisen.

Untersuchung auf Selektivität der antimuscarinischen Wirkung

Ziel:

Oxotremorin, ein spezifischer Agonist an muscarinischen Rezeptoren, erzeugt bei Ratten Läsionen der Magenschleimhaut und steigert die Speichelsekretion. Dieses Versuchsmodell wurde gewählt, um eine selektive Wirkung einer antimuscarinischen Substanz auf den Magen erkennen zu können.

Methodik:

Verwendet wurden 10 weibliche Albino-Ratten (Stamm Crl:COBS-CD (SD) BR) pro Behandlungsgruppe mit einem Körpergewicht von 120 bis 150 g, die bei freiem Zugang zu Trinkwasser 24 Stunden vor Versuchsbeginn ohne Nahrung waren.

Um in Vorversuchen die muscarinische Wirkung von Oxotremorin auf jedes der untersuchten Symptome zu ermitteln, wurde mit jeweils mindestens drei Dosen für jedes Symptom eine Dosis-Wirkungskurve erstellt.

Bei der Prüfung antimuscarinischer Substanzen wurde die Oxotremorin-Dosis gewählt, die in den Vorversuchen das zu beeinflussende Symptom bei 90 bis 100 % der Tiere ausgelöst hatte.

| | |
|---|---|
| Magenschleimhaut-Läsionen: | 0,62 mg/kg i.v. |
| Speichelsekretion: | 0,083 mg/kg i.v. |

Jede antimuscarinische Substanz wurde in gleichmäßig abgestuften Dosierungen 15 Minuten vor der Oxotremorin-Gabe intravenös verabreicht. Kontrollgruppen erhielten an Stelle der Prüfsubstanz das Lösungs- und Suspensionsmittel in entsprechender Menge.

Unmittelbar nach Oxotremorin-Gabe wurden die Tiere in einem Glaskäfig für 15 Minuten beobachtet.

Die Prüfung auf Beeinflussung der Oxotremorin-induzierten Speichelsekretion wurde blind durchgeführt, d.h. der Untersucher wußte nicht, wie die Tiere vorbehandelt waren.

Die Ergebnisse wurden als prozentuale Hemmung des Oxotremorin-Effektes (Prozentsatz der Tiere ohne das entsprechende Symptom) ausgedrückt. $ED_{50}$-Werte wurden nach der Methode von LITCHFIELD und WILCOXON (J. Pharmacol. Exp. Ther. 96, 99, 1949) ermittelt.

Die Effekte auf Schleimhaut-Läsionen des Magens wurden folgendermaßen bewertet:

Die Magenschleimhaut-Läsionen wurden erzeugt durch intravenöse Injektion von 0,62 mg/kg Oxotremorin 30 Minuten nach oraler Gabe von 1 mg/kg Neostigmin (Cholinesterase-Hemmer). 60 Minuten nach Neostigmin-Gabe wurden die Tiere getötet, die Mägen entnommen, geöffnet und auf Vorhandensein von Schleimhaut-Läsionen geprüft. Die schützende Wirkung der Prüfsubstanzen wurde als prozentuale Hemmung (Prozentsatz der Tiere ohne Läsionen) ausgedrückt. $ED_{50}$- bzw. $ED_{70}$-Werte wurden nach der Methode von LITCHFIELD und WILCOXON (s.o.) ermittelt.

Mydriasis

Der Effekt von Testsubstanzen auf die Pupillenweite von Ratten wurde folgendermaßen untersucht:

Die Substanzen wurden an Gruppen von jeweils 10 Tieren in wenigstens 3 gleichmäßig abgestuften Dosierungen intravenös appliziert. Die Pupillenweite wurde anschließend 10 Minuten lang auf evtl. Veränderungen (Auftreten von Mydriasis oder Miosis) beobachtet, und zwar wiederum blind, d.h. dem jeweiligen Untersucher war die Vorbehandlung der Tiere unbekannt. Es wurde der Prozentsatz der Versuchstiere ermittelt, bei denen eine Mydriasis auftrat. $ED_{50}$-Werte wurden wiederum nach LITCHFIELD und WILCOXON (s.o.) bestimmt.

2. Bindungsstudien an muscarinischen Rezeptoren: Bestimmung des $IC_{50}$-Wertes

Als Organspender dienten männliche Sprague-Dawley-Ratten mit 180-220 g Körpergewicht. Nach Entnahme von Herz, Magen und Großhirnrinde wurden alle weiteren Schritte im eiskalten Hepes-HCl-Puffer (pH 7,4; 100 m molar NaCl, 10 m molar $MgCl_2$) vorgenommen. Die glatte Muskulatur des Magenfundus wurde von der Magenschleimhaut freipräpariert und vorhomogenisiert. Das Gesamtherz wurde mit einer Schere zerkleinert. Alle Organe wurden abschließend in einem Potter homogenisiert.

Für den Bindungstest wurden die Organhomogenate in folgender Weise verdünnt:

| | |
|---|---|
| Glatter Muskel Magenfundus | 1:100 |
| Gesamtherz | 1:250 |
| Großhirnrinde | 1:3000 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30°C. Die Inkubationsdauer betrug 45 Minuten. Als Radioligand wurde 0,3 n molar $^{3}H$-N-Methylscopolamin ($^{3}H$-NMS) verwendet. Nach Beendigung der Inkubation durch Zentrifugation bei 14 000 g wurde die Radioaktivität im Pellet bestimmt. Sie repräsentiert die Summe von spezifischer und unspezifischer Bindung von $^{3}H$-NMS. Der Anteil der unspezifischen Bindung wurde definiert als jene Radioaktivität, die in Anwesenheit von 1 µ molar Chinuclidinylbenzilat gebunden wurde. Es wurden immer Vierfachbestimmungen vorgenommen. Die $IC_{50}$-Werte der nichtmarkierten Testsubstanzen wurden graphisch bestimmt. Sie repräsentieren jene Konzentration der Testsubstanz, bei welcher die spezifische Bindung von $^{3}H$-NMS an die muscarinischen Rezeptoren in den verschiedenen Organen um 50 % gehemmt wurde.

Nach den vorstehenden Angaben wurde beispielsweise die folgende Verbindung untersucht:

A = 4,9-Dihydro-4-/(1-methyl-4-piperidinyl)acetyl/-10H-thieno/3,4-b//1,5/benzodiazepin-10-on

Ergebnisse:

| Substanz | Rezeptor-Bindungs-Tests $IC_{50}$ [n Mol $l^{-1}$] | | Oxotremorin-Tests [µg/kg] i.v. | | | Mydriasis Pupillenweite (Ratte) |
|---|---|---|---|---|---|---|
| | Cortex | Glatter Muskel Magenfundus | Antiulcerogene Wirkung $ED_{50}$ | Antiulcerogene Wirkung $ED_{70}$ | Salivationshemmung $ED_{50}$ | $ED_{50}$ i.v. [µg/kg] |
| A | 20 | 75 | 7,8 | 14 | 21,5 | 30 |

Die Angaben in der vorstehenden Tabelle beweisen, daß die genannte Verbindung generell eine hohe Affinität zu muscarinischen Rezeptoren besitzt. Darüber hinaus kann man den Daten entnehmen, daß die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten aus der Großhirnrinde gegenüber solchen aus der glatten Muskulatur des Magens.

Aus den pharmakologischen Daten der vorstehenden Tabelle ergibt sich (in völliger Übereinstimmung mit den Rezeptor-Bindungs-Studien), daß die Entstehung Oxotremorin-induzierter Magenschleimhaut-Läsionen durch die genannten Verbindungen bereits bei Dosierungen gehemmt wird, bei denen noch keine Einschränkung der Speichelsekretion und keine Mydriasis beobachtet wird.

Die folgenden Beispiele dienen zur näheren Erläuterung der Erfindung. "F." bedeutet "Schmelzpunkt", "Z." bedeutet "Zersetzung".

## Beispiel 1

4,9-Dihydro-4-[(1-methyl-4-piperidinyl)acetyl]-10H-thieno[3,4-b][1,5]benzodiazepin-10-on

Eine Lösung von 2,2 g (0,01 Mol) 4,9-Dihydro-10H-thieno[3,4-b]-[1,5]benzodiazepin-10-on in 50 ml Dioxan und 10 ml Pyridin wurde bei Raumtemperatur mit einer Lösung von 0,02 Mol (1-Methyl-4-piperidinyl)acetylchlorid (hergestellt aus dem Kaliumsalz der 1-Methyl-4-piperidinessigsäure durch Umsetzung mit Thionylchlorid) in 100 ml Dioxan versetzt. Man erhitzte unter Rühren 3 Stunden bei 40°C und anschließend 2 Stunden bei 80°C. Nach Abgießen der Dioxanlösung wurde der schmierige Rückstand in Wasser aufgenommen, es wurde filtriert, das Filtrat wurde alkalisch gemacht und mit Methylenchlorid extrahiert. Der Extraktionsrückstand wurde säulenchromatographisch gereinigt (Kieselgel, Fließmittel:Essigsäureethylester+Methylenchlorid = 1+1; anschließend mit Fließmittel:Methylenchlorid+Cyclohexan+Methanol+Amoniak 102+23+23+3). Die gewünschte Fraktion wurde im Vakuum eingedampft, der Rückstand wurde aus Essigsäureethylester umkristallisiert.

F. 184-185°C,

Ausbeute: 28 % der Theorie.

$C_{19}H_{21}N_3O_2S$ (355,4)

| | | | | | | | | |
|---|---|---|---|---|---|---|---|---|
| Ber.: | C | 64,24 | H | 5,91 | N | 11,82 | S | 9,00 |
| Gef.: | | 64,26 | | 5,92 | | 12,02 | | 9,14 |

Entsprechend erhielt man:

aus 4,9-Dihydro-1-methyl-10H-thieno[3,4-b][1,5]benzodiazepin-10-on und (1-Methyl-4-piperidinyl)acetylbromid das 4,9-Dihydro-1-methyl-4-[(1-methyl-4-piperidinyl)acetyl]-10H-thieno[3,4-b][1,5]benzodiazepin-10-on,

aus 4,9-Dihydro-3-methyl-10H-thieno[3,4-b][1,5]benzodiazepin-10-on und (1-Methyl-4-piperidinyl)acetylchlorid das 4,9-Dihydro-3-methyl-4-[(1-methyl-4-piperidinyl)acetyl]-10H-thieno[3,4-b]/1,5/benzodiazepin-10-on; F.: 192-193°C (Acetonitril);

aus 4,9-Dihydro-1,3-dimethyl-10H-thieno[3,4-b][1,5]benzodiazepin-10-on und (1-Methyl-4-piperidinyl)acetylchlorid das 4,9-Dihydro-1,3-dimethyl-4-[(1-methyl-4-piperidinyl)acetyl]-10H-thieno[3,4-b][1,5]benzodiazepin-10-on;

aus 3-Chlor-4,9-dihydro-10H-thieno[3,4-b][1,5]benzodiazepin-10-on und (1-Methyl-4-piperidinyl)acetylchlorid das 3-Chlor-4,9-dihydro-4-[(1-methyl-4-piperidinyl)acetyl]-10H-thieno[3,4-b][1,5]benzodiazepin-10-on;

aus 4,9-Dihydro-10H-thieno[3,4-b][1,5]benzodiazepin-10-on und (1-Methyl-1,2,5,6-tetrahydro-4-pyridinyl)acetylchlorid das 4,9-Dihydro-4-[(1-methyl-1,2,5,6-tetrahydro-4-pyridinyl)-acetyl]-10H-thieno[3,4-b][1,5]benzodiazepin-10-on;

aus 4,9-Dihydro-10H-thieno[3,4-b][1,5]benzodiazepin-10-on und (1-Methyl-4-piperidinyliden)acetylchlorid das 4,9-Dihydro-4-[(1-methyl-4-piperidinyliden)acetyl]-10H-thieno-[3,4-b][1,5]benzodiazepin-10-on;

aus 4,9-Dihydro-10H-thieno[3,4-b][1,5]benzodiazepin-10-on
und 1-Methyl-1,2,5,6-tetrahydro-isonicotinsäurechlorid-hydrochlorid
das 4,9-Dihydro-4-[(1-methyl-1,2,5,6-tetrahydro-4-pyridinyl)-carbonyl]-10H-thieno[3,4-b][1,5]benzodiazepin-10-on
vom F. 210-212°C (Acetonitril);

aus 4,9-Dihydro-1-methyl-10H-thieno[3,4-b][1,5]benzodiazepin-10-on
und 1-Methyl-1,2,5,6-tetrahydro-isonicotinsäurechlorid-hydrochlorid
das 4,9-Dihydro-1-methyl-4-[(1-methyl1,2,5,6-tetrahydro-4-pyridinyl)carbonyl]-10H-thieno[3,4-b][1,5]benzodiazepin-10-on;

aus 4,9-Dihydro-3-methyl-10H-thieno[3,4-b][1,5]benzodiazepin-10-on
und 1-Methyl-1,2,5,6-tetrahydro-isonicotinsäurechlorid-hydrochlorid (H. Leditschke, Arch. Pharm. 295, 328 [1962])
das 4,9-Dihydro-3-methyl-4-[(1-methyl-1,2,5,6-tetrahydro-4-pyridinyl)carbonyl]-10H-thieno[3,4-b][1,5]benzodiazepin-10-on;

aus 4,9-Dihydro-1,3-dimethyl-10H-thieno[3,4-b][1,5]benzodiazepin-10-on
und 1-Methyl-1,2,5,6-tetrahydro-isonicotinsäurechlorid-hydrochlorid das
4,9-Dihydro-1,3-dimethyl-4-[(1-methyl-1,2,5,6-tetrahydro-4-pyridinyl)carbonyl]-10H-thieno[3,4-b][1,5]benzodiazepin-10-on;

aus 3-Chlor-4,9-dihydro-10H-thieno[3,4-b][1,5]benzodiazepin-10-on
und 1-Methyl-1,2,5,6-tetrahydro-isonicotinsäurechlorid-hydrochlorid das
3-Chlor-4,9-dihydro-4-[(1-methyl-1,2,5,6-tetrahydro-4-pyridinyl)carbonyl]-10H-thieno[3,4-b][1,5]benzodiazepin-10-on;

aus 4,9-Dihydro-10H-thieno[3,4-b][1,5]benzodiazepin-10-on
und (8-Methyl-8-azabicyclo[3,2,1]oct-3-yl)acetylchlorid das
Diastereomerengemisch von
4,9-Dihydro-4-[(8-methyl-8-azabicyclo[3,2,1]oct-3-yl)-acetyl]-10H-thieno[3,4-b][1,5]benzodiazepin-10-on;

aus 4,9-Dihydro-1-methyl-10H-thieno[3,4-b][1,5]benzodiazepin-10-on
und (8-Methyl-8-azabicyclo[3,2,1]oct-3-yl)acetylchlorid das
Diasteremoerengemisch von
4,9-Dihydro-1-methyl-4-[(8-methyl-8-azabicyclo[3,2,1]-oct-3-yl)acetyl]-10H-thieno[3,4-b][1,5]benzodiazepin-10-on;

aus 4,9-Dihydro-3-methyl-10H-thieno[3,4-b][1,5]benzodiazepin-10-on
und (8-Methyl-8-azabicyclo[3,2,1]oct-3-yl)acetylchlorid das
Diastereomerengemisch von
4,9-Dihydro-3-methyl-4-[(8-methyl-8-azabicyclo[3,2,1]-oct-3-yl)acetyl]-10H-thieno[3,4-b][1,5]benzodiazepin-10-on;

aus 4,9-Dihydro-1,3-dimethyl-10H-thieno[3,4-b][1,5]benzodiazepin-10-on
und (8-Methyl-8-azabicyclo[3,2,1]oct-3-yl)acetylchlorid das
Diastereomerengemisch von
4,9-Dihydro-1,3-dimethyl-4-[(8-methyl-8-azabicyclo[3,2,1]-oct-3-yl)acetyl]-10H-thieno[3,4-b][1,5]benzodiazepin-10-on;

aus 3-Chlor-4,9-dihydro-10H-thieno[3,4-b][1,5]benzodiazepin-10-on
und (8-Methyl-8-azabicyclo[3,2,1]oct-3-yl)acetylchlorid das
Diastereomerengemisch von
3-Chlor-4,9-dihydro-4-[(8-methyl-8-azabicyclo[3,2,1]oct-3-yl)acetyl]-10H-thieno[3,4-b][1,5]benzodiazepin-10-on.

Beispiel 2

4,9-Dihydro-4-[(1-methyl-1,2,5,6-tetrahydro-4-pyridinyl)carbonyl]-10H-thieno[3,4-b][1,5]benzodiazepin-10-on

a) Ein Gemisch von 12,9 g (0,06 Mol) 4,9-Dihydro-10H-thieno[3,4-b][1,5]benzodiazepin-10-on, 11,7 g (0,066 Mol Isonicotinsäurechlorid-hydrochlorid, 10,3 g Pyridin (0,13 Mol) und 250 ml Dioxan wurde 6 Stunden unter Rückfluß erhitzt. Nach Abdekantieren vom Lösungsmittel wurde der schmierige Rückstand in Wasser aufgenommen, es wurde filtriert. Das Filtrat wurde mit Natriumcarbonat alkalisch gemacht. Es schied sich das 4,9-Dihydro-4-isonicotinoyl-10H-thieno[3,4-b][1,5]benzodiazepin-10-on ab, das nach dem Umkristallisieren aus Essigsäureethylester bei 282-284°C schmolz. Ausbeute 40 % der Theorie.

b) 2,4 g (0,0074 Mol) 4,9-Dihydro-4-isonicotinoyl-10H-thieno[3,4-b][1,5]benzodiazepin-10-on wurden in 30 ml Dimethylformamid gelöst. Nach der Zugabe von 2 ml Methyljodid wurde 3 Stunden bei Raumtemperatur gerührt. Dann wurde im Vakuum eingedampft. Man erhielt 3,4 g des Methoiodids vom F. 295°C(Z.)

c) 3,4 g (0,0073 Mol) 4,9 Dihydro-4-isonicotinoyl-10H-thieno[3,4-b][1,5]benzodiazepin-10-on-methoiodid wurden in 40 ml Methanol suspendiert und bei 0°C mit 0,5 g (0,014 Mol) Natriumborhydrid portionsweise versetzt. Nach beendeter Wasserstoffentwicklung wurde noch 2 Stunden im Eisbad nachgerührt. Dann versetzte man mit 100 ml Eiswasser, filtrierte und extrahierte das Filtrat mit Methylenchlorid. Der Extraktionsrückstand wurde aus Acetonitril umkristallisiert. Man erhielt das 4,9-Dihydro-4-[(1-methyl-1,2,5,6-tetrahydro-4-pyridinyl)carbonyl]-10H-thieno[3,4-b][1,5]benzodiazepin-10-on vom F. 210-212°C in einer Ausbeute von 35 % der Theorie.

Die erhaltene Base wurde in Ethylacetat gelöst und mit einer Lösung von Chlorwasserstoff in Dioxan versetzt. Das ausgefallene Hydrochlorid wurde aus Ethanol umkristallisiert; F.: 215-217°C (Z.).

Entsprechend erhielt man:

aus 4,9-Dihydro-10H-thieno[3,4-b][1,5]benzodiazepin-10-on
und 4-Pyridinessigsäurechlorid-hydrochlorid über
4,9-Dihydro-4-[(4-pyridinyl)acetyl]-10H-thieno[3,4-b][1,5]-benzodiazepin-10-on
und 4,9-Dihydro-4-[(4-pyridinyl)acetyl]-10H-thieno[3,4-b][1,5]-benzodiazepin-10-on-methoiodid
das 4,9-Dihydro-4-[(1-methyl-1,2,5,6-tetrahydro-4-pyridinyl)-acetyl]-10H-thieno[3,4-b][1,5]benzodiazepin-10-on;

aus 4,9-Dihydro-1-methyl-10H-thieno[3,4-b][1,5]benzodiazepin-10-on
und Isonicotinsäurechlorid-hydrochlorid über
4,9-Dihydro-1-methyl-4-[(4-pyridinyl)carbonyl]-10H-thieno-[3,4-b][1,5]benzodiazepin-10-on
und 4,9-Dihydro-1-methyl-4-[(4-pyridinyl)carbonyl]-10H-thieno-[3,4-b][1,5]benzodiazepin-10-on-methoiodid
das 4,9-Dihydro-1-methyl-4-[(1-methyl-1,2,5,6-tetrahydro-4-pyridinyl)carbonyl]-10H-thieno[3,4-b][1,5]benzodiazepin-10-on;

aus 4,9-Dihydro-3-methyl-10H-thieno[3,4-b][1,5]benzodiazepin-10-on
und Isonicotinsäurechlorid-hydrochlorid über
4,9-Dihydro-3-methyl-4-[(4-pyridinyl)carbonyl]-10H-thieno-[3,4-b][1,5]benzodiazepin-10-on
und 4,9-Dihydro-3-methyl-4-[(4-pyridinyl)carbonyl]-10H-thieno-[3,4-b][1,5]benzodiazepin-10-on-methoiodid
das 4,9-Dihydro-3-methyl-4-[(1-methyl-1,2,5,6-tetrahydro-4-pyridinyl)carbonyl]-10H-thieno[3,4-b][1,5]benzodiazepin-10-on;

aus 4,9-Dihydro-1,3-dimethyl-10H-thieno[3,4-b][1,5]benzodiazepin-10-on
und Isonicotinsäurechlorid-hydrochlorid über 4,9-Dihydro-1,3-dimethyl-4-[(4-pyridinyl)carbonyl]-10H-thieno[3,4-b][1,5]benzodiazepin-10-on
und 4,9-Dihydro-1,3-dimethyl-4-[(4-pyridinyl)carbonyl]-10H-thieno[3,4-b][1,5]benzodiazepin-10-on-methoiodid
das 4,9-Dihydro-1,3-dimethyl-4-[(1-methyl-1,2,5,6-tetrahydro-4-pyridinyl)carbonyl]-10H-thieno[3,4-b][1,5]benzodiazepin-10-on;

aus 3-Chlor-4,9-dihydro-10H-thieno[3,4-b][1,5]benzodiazepin-10-on
und Isonicotinsäurechlorid-hydrochlorid über 3-Chlor-4,9-dihydro-4-[(4-pyridinyl)carbonyl]-10H-thieno-[3,4-b][1,5]benzodiazepin-10-on
und 3-Chlor-4,9-dihydro-4-[(4-pyridinyl)carbonyl]-10H-thieno-[3,4-b][1,5]benzodiazepin-10-on-methoiodid
das 3-Chlor-4,9-dihydro-4-[(1-methyl-1,2,5,6-tetrahydro-4-pyridinyl)carbonyl]-10H-thieno[3,4-b][1,5]benzodiazepin-10-on.

## Beispiel 3

**4,9-Dihydro-4-[(1-methyl-4-piperidinyl)acetyl]-10H-thienobenzodiazepin-10-on**

Man erwärmte ein Gemisch von 0,983 g 1-Methyl-4-piperidinyl-essigsäure (6,25 mMol) und 0,2o g (6,25 mMol) 75 proz. Natriumhydrid (in Paraffinöl) in 16 ml Dimethylformamid bei 50-80°C so lange, bis die Wasserstoffentwicklung beendet war (2 bis 3 Stunden). Zu dem gebildeten Natriumsalz der Säure fügte man

1,35 g (6,24 mMol) 4,9-Dihydro-10H-thieno/3,4-b/ /1,5/benzodiazepin-10-on und tropfte bei -10°C 0,99 g 98 % Phosphoroxidchlorid in 10 Minuten zu. Man rührte 4 Stunden bei -10°C, 4 Stunden bei 0°C und 20 Stunden bei Raumtemperatur. Man goß den Ansatz auf Eis, stellte mit Natronlauge auf pH 3,5, schüttelte mit Methylenchlorid aus, stellte die wässrige Phase auf pH 9 und schüttelte wieder mit Methylenchlorid aus. Man wusch die organische Phase mit Wasser und engte sie im Vakuum ein. Man erhielt 0,59 g (27 % der Theorie) an 4,9-Dihydro-4-/(1-methyl-4-piperidinyl)acetyl/-10H-thieno/3,4-b/ /1,5/benzodiazepin-10-on vom F. 184-185°C (Essigsäureethylester).

Entsprechend erhielt man:

aus 4,9-Dihydro-10H-thieno/3,4-b/ /1,5/benzodiazepin-10-on und (1-Methyl-4-piperidinyliden)essigsäure das 4,9-Dihydro-4-/(1-methyl-4-piperidinyliden)acetyl/-10H-thieno/3,4-b/ /1,5/benzodiazepin-10-on;

aus 4,9-Dihydro-10H-thieno/3,4-b/ /1,5/benzodiazepin-10-on und 1-Methyl-1,2,5,6-tetrahydro-4-pyridinessigsäure das 4,9-Dihydro-4-/(1-methyl-1,2,5,6-tetrahydro-4-pyridinyl)-acetyl/-10H-thieno/3,4-b/ /1,5/benzodiazepin-10-on;

aus 4,9-Dihydro-10H-thieno/3,4-b/ /1,5/benzodiazepin-10-on und 1-Methyl-1,2,5,6-tetrahydro-isonicotinsäure das 4,9-Dihydro-4-/(1-methyl-1,2,5,6-tetrahydro-4-pyridinyl)-carbonyl/-10H-thieno/3,4-b/ /1,5/benzodiazepin-10-on vom F. 210-212°C (Acetonitril);

aus 4,9-Dihydro-1-methyl-10H-thieno/3,4-b/ /1,5/benzodiazepin-10-on
und (1-Methyl-4-piperidinyl)essigsäure das 4,9-Dihydro-1-methyl-4-/(1-methyl-4-piperidinyl)acetyl/-10H-thieno/3,4-b/ /1,5/benzodiazepin-10-on;

0086982

aus 4,9-Dihydro-10H-thieno/3,4-b7/1,57benzodiazepin-10-on und (1,3-Dimethyl-4-piperidinyl)essigsäure das 4,9-Dihydro-4-/(1,3-dimethyl-4-piperidinyl)acetyl7-10H-thieno/3,4-b7/1,57benzodiazepin-10-on (Gemisch von 2 Diastereomeren);

aus 4,9-Dihydro-10H-thieno/3,4-b7/1,57benzodiazepin-10-on und (8-Methyl-8-azabicyclo/3,2,17oct-3-yl)essigsäure das 4,9-Dihydro-4-/(8-methyl-8-azabicyclo/3,2,17oct-3-yl)-acetyl7-10H-thieno/3,4-b7/1,57benzodiazepin-10-on (Gemisch von 2 Diastereomeren).

Beispiel 4

4,9-Dihydro-4-/(1-methyl-4-piperidinyl)acetyl7-10H-thieno-/3,4-b7/1,57benzodiazepin-10-on

Zu einer Suspension von 1,57 g (10 mMol) (1-Methyl-4-piperidinyl)-essigsäure in 20 ml Tetrahydrofuran wurden bei 0°C 1,1 g Chlorameisensäureethylester zugetropft. Man fügte 2,16 g (10 mMol) 4,9-Dihydro-10H-thieno/3,4-b7/1,57benzodiazepin-10-on zu der entstandenen Suspension, rührte noch eine Stunde bei 0°C und anschließend 4 Stunden bei Raumtemperatur. Man goß auf 160 ml 2 N Natronlauge, extrahierte mit Toluol und engte die organische Phase zur Trockne ein. Nach säulenchromatographischer Reinigung (Kieselgel; Dioxan/Methanol 1:1) erhielt man 4,9-Dihydro-4-/(1-methyl-4-piperidinyl)-acetyl7-10H-thieno-/3,4-b7/1,57benzodiazepin-10-on, F. 184-185°C (Essigsäureethylester), Ausbeute: 0,84 g (24 % der Theorie).

Analog erhielt man:

aus 4,9-Dihydro-10H-thieno[3,4-b][1,5]benzodiazepin-10-on und 1-Methyl-1,2,5,6-tetrahydro-isonicotinsäure das 4,9-Dihydro-4-[(1-methyl-1,2,5,6-tetrahydro-4-pyridinyl)-carbonyl]-10H-thieno[3,4-b][1,5]benzodiazepin-10-on vom F. 210-212°C (Acetonitril).

## Beispiel 5

**endo-4,9-Dihydro-4-[(8-methyl-8-azabicyclo[3,2,1]oct-3-yl)-acetyl]-10H-thieno[3,4-b][1,5]benzodiazepin-10-on**

Hergestellt analog Beispiel 1 aus 4,9-Dihydro-10H-thieno[3,4-b]-[1,5]benzodiazepin-10-on und (endo-8-Methyl-8-azabicyclo-[3,2,1]oct-3-yl)acetylchlorid in einer Ausbeute von 14 % der Theorie. Farblose Kristalle vom F. 167-168°C (Acetonitril).

$C_{21}H_{23}N_3O_2S$ (381,49)

| | | | | | | | | |
|---|---|---|---|---|---|---|---|---|
| Ber.: | C | 66,12 | H | 6,08 | N | 11,01 | S | 8,40 |
| Gef.: | | 66.16 | | 5,93 | | 11,18 | | 8,47 |

IR ($CH_2Cl_2$): NH 3365/cm; CO 1665/cm (breit)
UV (Ethanol): Schulter bei 250 nm (E = 0,15)
UV (Ethanol/KOH): Schulter bei 252 nm (E = 0,16); Schulter bei 284 nm (E = 0,08) (c = 50 mg/l, Schichtdicke: 2 mm)
$^1$H-NMR ($CDCl_3/D_2O$): $\delta$ = 8,09 (1H-d; J=2,4 Hz; 1-H); 7,0-7,5 (5H-m; ar. H); 2,8-3,2 (2H-m); 2,4-2,7 (2H-m); 1,7-2,3 (5H-m); 2,18 (3H-s; N-$CH_3$); 0,8-1,5 ppm (4H-m)

Im folgenden wird die Herstellung pharmazeutischer Anwendungsformen anhand einiger Beispiele beschrieben:

Beispiel I

Tabletten mit 5 mg 4,9-Dihydro-4-/(1-methyl-4-piperidinyl)-acetyl/-10H-thieno/3,4-b//1,5/benzodiazepin-10-on

Zusammensetzung:

1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45°C getrocknet, nochmals durch obiges Sieb gerieben, mit Magnesiumstearat vermischt und zu Tabletten verpreßt.

Tablettengewicht: 220 mg

Stempel: 9 mm

Beispiel II

Dragées mit 5 mg 4,9-Dihydro-4-/(1-methyl-4-piperidinyl)-acetyl/-10H-thieno/3,4-b//1,5/benzodiazepin-10-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 300 mg

Beispiel III

Ampullen mit 1 mg 4,9-Dihydro-4-[(1-methyl-1,2,5,6-tetrahydro-4-pyridinyl)carbonyl]-10H-thieno[3,4-b][1,5]benzodiazepin-10-on-hydrochlorid

Zusammensetzung:
1 Ampulle enthält:

| | | |
|---|---|---|
| Wirkstoff | | 1,0 mg |
| Natriumchlorid | | 8,0 mg |
| Dest. Wasser | ad | 1 ml |

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1ml-Ampullen abgefüllt. Sterilisation: 20 Minuten bei 120°C.

Beispiel IV

Suppositorien mit 5 mg 4,9-Dihydro-4-[(1-methyl-4-piperidinyl)-acetyl]-10H-thieno[3,4-b][1,5]benzodiazepin-10-on

Zusammensetzung:
1 Zäpfchen enthält:

| | |
|---|---|
| Wirkstoff | 5,0 mg |
| Zäpfchenmasse (z.B. Witepsol W 45 R) | 1 695,0 mg |
| | 1 700,0 mg |

Herstellungsverfahren:

Die feinpulverisierte Wirksubstanz wird in der geschmolzenen und auf 40°C abgekühlten Zäpfchenmasse suspendiert. Man gießt die Masse bei 37°C in leicht vorgekühlte Zäpfchenformen aus. Zäpfchengewicht: 1,7 g

Beispiel V

Tropfen mit 4,9-Dihydro-4-[(1-methyl-1,2,5,6-tetrahydro-4-pyridinyl)carbonyl]-10H-thieno[3,4-b][1,5]benzodiazepin-10-on-hydrochlorid

Zusammensetzung:
100 ml Tropflösung enthalten:

| | | |
|---|---|---|
| p-Hydroxybenzoesäuremethylester | | 0,035 g |
| p-Hydroxybenzoesäurepropylester | | 0,015 g |
| Anisöl | | 0,05 g |
| Menthol | | 0,06 g |
| Ethanol rein | | 10,0 g |
| Wirkstoff | | 0,5 g |
| Natriumcyclamat | | 1,0 g |
| Glycerin | | 15,0 g |
| Dest. Wasser | ad | 100,0 ml |

Herstellungsverfahren:

Die Wirksubstanz und Natriumcyclamat werden in ca. 70 ml Wasser gelöst und Glycerin zugefügt. Man löst die p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

Patentansprüche

1.) Substituierte Thienobenzodiazepinone der allgemeinen Formel

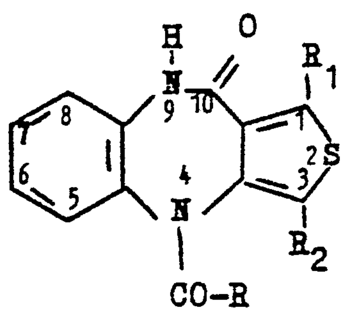

(I),

in der

$R_1$ ein Wasserstoffatom oder eine Alkylgruppe mit 1 bis 4 Kohlenstoffatomen,

$R_2$ ein Wasserstoff- oder Halogenatom oder eine Alkylgruppe mit 1 bis 4 Kohlenstoffatomen und

R eine, gegebenenfalls im heterocyclischen Ring durch eine weitere Methylgruppe substituierte (1-Methyl-4-piperidinyl)methyl-, (1-Methyl-1,2,5,6-tetrahydro-4-pyridinyl)methyl-, 1-Methyl-1,2,5,6-tetrahydro-4-pyridinyl-, (1-Methyl-4-piperidinyliden)methyl- oder (8-Methyl-8-azabicyclo[3,2,1]oct-3-yl)methylgruppe bedeuten

gegebenenfalls ihre geometrischen Isomeren und Enantiomeren und ihre physiologisch verträglichen Säureadditionssalze mit anorganischen oder organischen Säuren.

2.) Substituierte Thienobenzodiazepinone der allgemeinen Formel I gemäß Anspruch 1, dadurch gekennzeichnet, daß $R_1$ und $R_2$, die gleich oder voneinander verschieden sind, ein Wasserstoffatom oder die Methylgruppe, und R eine (1-Methyl-4-piperidinyl)-methyl-, 1-Methyl-1,2,5,6-tetrahydro-4-pyridinyl- oder (8-Methyl-8-azabicyclo-[3,2,1]oct-3-yl)methylgruppe bedeuten, gegebenenfalls ihre geometrischen Isomeren und Enantiomeren und ihre physiologisch verträglichen Säureadditionssalze mit anorganischen oder organischen Säuren.

3.) 4,9-Dihydro-4-[(1-methyl-4-piperidinyl)acetyl]-10H-thieno-[3,4-b][1,5]benzodiazepin-10-on und dessen physiologisch verträgliche Säureadditionssalze mit anorganischen oder organischen Säuren.

4.) Arzneimittel enthaltend eine oder mehrere Verbindungen der allgemeinen Formel I gemäß Anspruch 1, 2 oder 3 neben den üblichen Träger- und/oder Hilfsstoffen.

5.) Verfahren zur Herstellung neuer, substituierter Thienobenzodiazepinone der allgemeinen Formel

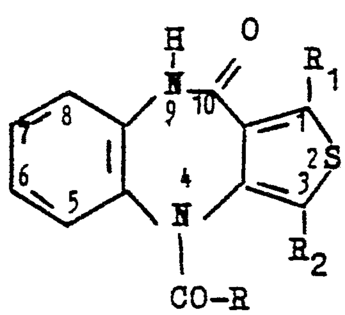

(I),

in der

$R_1$ ein Wasserstoffatom oder eine Alkylgruppe mit 1 bis 4 Kohlenstoffatomen,

$R_2$ ein Wasserstoff- oder Halogenatom oder eine Alkylgruppe mit 1 bis 4 Kohlenstoffatomen und

R eine, gegebenenfalls im heterocyclischen Ring durch eine weitere Methylgruppe substituierte (1-Methyl-4-piperidinyl)methyl-, (1-Methyl-1,2,5,6-tetrahydro-4-pyridinyl)-methyl-, 1-Methyl-1,2,5,6-tetrahydro-4-pyridinyl-, (1-Methyl-4-piperidinyliden)methyl- oder (8-Methyl-8-azabicyclo/3,2,1/oct-3-yl)methylgruppe bedeuten,

und von deren Säureadditionssalzen mit anorganischen oder organischen Säuren dadurch gekennzeichnet, daß

a) Thienobenzodiazepinone der allgemeinen Formeel

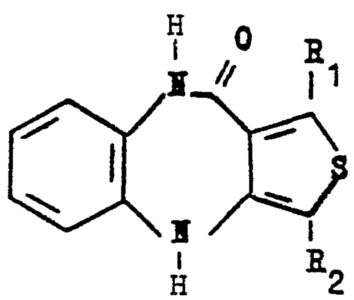

(II),

in der $R_1$ und $R_2$ wie oben definiert sind, mit Säurederivaten der allgemeinen Formel

$$Z - \underset{\underset{O}{\parallel}}{C} - R \qquad (III),$$

in der R wie oben definiert ist und Z eine nucleofuge Gruppe bedeutet, in einem inerten Lösungsmittel bei Temperaturen zwischen -25°C und +130°C acyliert werden oder

b) zur Herstellung von Verbindungen der allgemeinen Formel I, in der R eine gegebenenfalls im heterocyclischen Ring durch eine weitere Methylgruppe substituierte (1-Methyl-1,2,5,6-tetrahydro-4-pyridinyl)methyl- oder 1-Methyl-1,2,5,6-tetrahydro-4-pyridinylgruppe bedeutet, ein Pyridiniumsalz der allgemeinen Formel

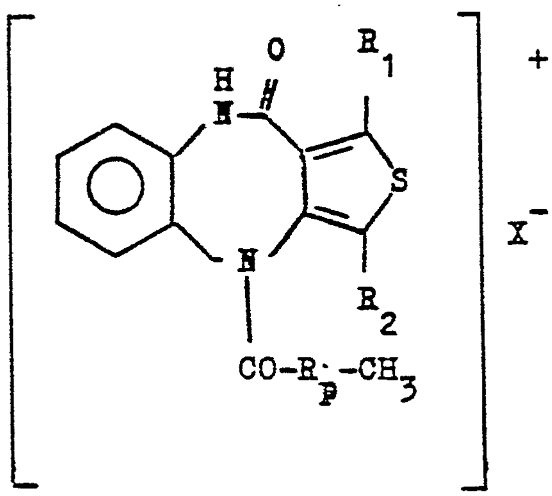

(Va),

in der $R_1$ und $R_2$ wie oben definiert sind, X den Säurerest einer starken Sauerstoffsäure oder ein Halogenatom und $R_p$ eine gegebenenfalls methylsubstituierte 4-Pyridinyl- oder (4-Pyridinyl)methylgruppe bedeuten, mit Borhydriden oder Alkoxyborhydriden in protischen Lösungsmitteln bei Temperaturen zwischen -40°C und +50°C reduziert wird, und gegebenenfalls so erhaltene Verbindungen der allgemeinen Formel I anschließend in ihre Salze mit anorganischen oder organischen Säuren übergeführt werden.

6.) Verfahren gemäß Anspruch 5a, dadurch gekennzeichnet, daß als Säurederivate der allgemeinen Formel III ein Säurehalogenid, ein Ester, ein Säureanhydrid, ein gemischtes Säureanhydrid oder ein N-Alkyl-2-acyloxypyridiniumsalz verwendet wird.

7.) Verfahren gemäß Anspruch 5a, dadurch gekennzeichnet, daß als Säurederivat der allgemeinen Formel III das gemischte Anhydrid mit einer starken Mineralsäure, insbesondere mit der Dichlorphosphorsäure, verwendet und die Reaktion in einem inerten Lösungsmittel in Gegenwart eines säurebindenden Mittels durchgeführt wird.

8.) Verfahren gemäß Anspruch 5b, dadurch gekennzeichnet, daß ein Pyridiniumsalz der allgemeinen Formel Va mittels Natrium- oder Kaliumtetrahydridoborat oder Natrium- oder Kaliumalkoxy-, dialkoxy- oder trialkoxyborhydriden bei -5 bis +10°C in Anwesenheit eines protischen Lösungsmittels, vorzugsweise von Wasser, Methanol, Ethanol oder 2-Propanol oder von Gemischen dieser Lösungsmittel, reduziert wird.

9.) Verfahren zur Herstellung der Verbindungen der allgemeinen Formel Va gemäß Anspruch 6b, dadurch gekennzeichnet, daß ein Thienobenzodiazepinon der allgemeinen Formel

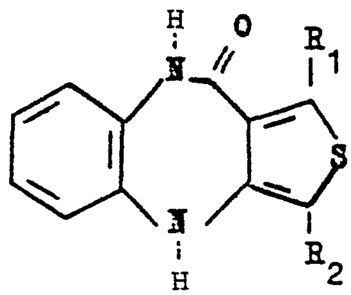

(II),

in der $R_1$ und $R_2$ wie oben erwähnt definiert sind, mit einem Acylierungsmittel der allgemeinen Formel

$$Z - \underset{\overset{\|}{O}}{C} - R_p \qquad (IV),$$

in der Z eine nucleofuge Gruppe, vorzugsweise ein Chlor-, Brom- oder Jodatom, und $R_p$ eine gegebenenfalls methylsubstituierte 4-Pyridinyl- oder (4-Pyridinyl)methylgruppe darstellen, in einem inerten Lösungsmittel bei Temperaturen bis zum Siedepunkt des Reaktionsgemisches zu einer Verbindung der allgemeinen Formel

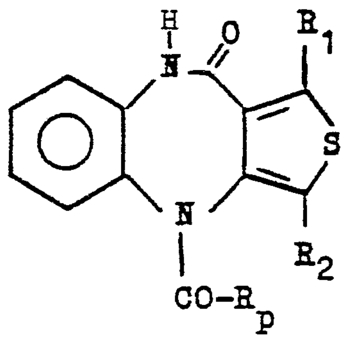

(V),

umgesetzt wird, und anschließend eine so erhaltene Verbindung mit einem Methylierungsmittel der allgemeinen Formel

$$H_3C - X \qquad (VI),$$

in der X den Säurerest einer starken Sauerstoffsäure oder ein Halogenatom bedeutet, in einem inerten Lösungsmittel bei Temperaturen zwischen $-20^{\circ}$ und $+130^{\circ}C$ zu der entsprechenden Verbindung der allgemeinen Formel Va methyliert wird.

Europäisches Patentamt

# EUROPÄISCHER RECHERCHENBERICHT

0086982
Nummer der Anmeldung

EP 83 10 0680

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,A | EP-A-0 039 519 (BYK GULDEN LOMBERG CHEMISCHE FABRIK)<br>* Ansprüche 1, 7 *<br>--- | 1,6 | C 07 D 495/04<br>C 07 D 519/00<br>A 61 K 31/55 //<br>(C 07 D 495/04<br>C 07 D 333/00<br>C 07 D 243/00 )<br>(C 07 D 519/00<br>C 07 D 495/00<br>C 07 D 451/00 ) |
| D,A | DE-A-1 795 176 (DR. KARL THOMAE)<br>----- | | |
| | | | **RECHERCHIERTE SACHGEBIETE (Int. Cl. 3)** |
| | | | A 61 K 31/55<br>C 07 D 495/04<br>C 07 D 519/00 |

Der vorliegende Recherchenbericht wurde für alle Patentansprüche erstellt.

| Recherchenort | Abschlußdatum der Recherche | Prüfer |
|---|---|---|
| BERLIN | 02-05-1983 | PHILLIPS N.G.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument